**Europäisches Patentamt**

**European Patent Office**

**Office européen des brevets**

(19)

(11) Publication number: **0 106 356**
**B1**

(12) # EUROPEAN PATENT SPECIFICATION

(45) Date of publication of patent specification: **19.11.87**

(21) Application number: **83110375.9**

(22) Date of filing: **18.10.83**

(51) Int. Cl.⁴: **C 07 C 17/158,**
**C 07 C 19/045, B 01 J 29/34,**
**B 01 J 29/04**

(54) Catalytic process for producing saturated dihalohydrocarbons.

(30) Priority: **18.10.82 US 434995**

(43) Date of publication of application:
**25.04.84 Bulletin 84/17**

(45) Publication of the grant of the patent:
**19.11.87 Bulletin 87/47**

(84) Designated Contracting States:
**BE DE FR GB IT NL SE**

(56) References cited:
**WO-A-80/02023**
**DE-A-1 543 241**
**DE-A-2 426 587**
**GB-A-1 263 806**
**GB-A-2 095 242**
**GB-A-2 095 245**
**US-A-3 720 723**
**US-A-3 987 118**
**US-A-4 170 571**
**US-A-4 276 438**

**The file contains technical information**
**submitted after the application was filed and**
**not included in this specification**

(73) Proprietor: **THE DOW CHEMICAL COMPANY**
**2030 Dow Center Abbott Road P.O. Box 1967**
**Midland, MI 48640 (US)**

(72) Inventor: **Diesen, Ronald Willian**
**5802 Flaxmoor**
**Midland Michigan 48640 (US)**

(74) Representative: **Weickmann, Heinrich, Dipl.-Ing.**
**et al**
**Patentanwälte Dipl.-Ing. H.Weickmann Dipl.-**
**Phys.Dr. K.Fincke Dipl.-Ing. F.A.Weickmann**
**Dipl.-Chem. B. Huber Dr.-Ing. H. Liska Dipl.-**
**Phys.Dr. J. Prechtel Postfach 860820**
**D-8000 München 86 (DE)**

(56) References cited:
**"ULLMANNS ENCYKLOPÄDIE DER**
**TECHNISCHEN CHEMIE", 4th edition, vol. 17,**
**1979, VERLAG CHEMIE, Weinheim, "Milchsäure**
**bis Petrolkoks", pages 9-18**

EP 0 106 356 B1

Courier Press, Leamington Spa, England.

**Description**

This invention relates to a catalytic chlorination process. More specifically, it relates to specific catalysts for use in the oxychlorination of chlorinated hydrocarbons.

1,2-Dichloroethane (ethylene dichloride) is useful in that it may be dehydrohalogenated to vinyl chloride. 1,2-Dichloroethane has been prepared in the past by the chlorination of ethene using molecular chlorine and by the catalytic oxychlorination of ethene. Several oxychlorination catalysts are known. For example, USP 3,892,816 describes the catalytic oxychlorination of ethene by contacting ethene, hydrogen chloride, and molecular oxygen at a temperature of from about 450°F (232°C) to about 670°F (354°C) in the presence of a supported, stabilized cupric chloride catalyst. USP 3,634,330 discloses a supported oxychlorination catalyst composed of mixtures of cupric chloride and at least one alkali metal chloride or alkaline earth metal chloride.

US—A—4276438 discloses a process for conversion of aromatic compounds to dialkylbenzene compounds rich in the p-dialkylbenzene isomer. The process employs a composite catalyst which includes a particular type of zeolites modified by treatment with a compound derived from one or more elements of Group 1B, i.e., Cu, Ag and Au.

The composite catalyst is prepared by ion-exchanging the original alkali metal of the zeolitic support with Cu, Ag and/or Au. In a particular embodiment, a copper compound ion-exchanged in the zeolite is converted to copper oxide.

Similarly, US—A—4170571 discloses a combustion catalyst which is prepared by ion-exchanging a ZSM-5 type zeolite with divalent copper cations and subjecting the resulting composition to rigorous oxidation to convert incorporated copper to copper oxide.

GB—A—2095242 discloses a catalyst composition comprising metallic silver and/or a compound thereof and one or more compounds of manganese, cobalt or nickel. The specially preferred catalyst is identified by X-ray defraction as $AgMn_2O_4$ and $AgCoO_2$, which is prepared by co-precipitation of the metal nitrates with subsequent calcination.

GB—A—2095245 uses zeolite catalysts preferably partially or wholly exchanged with one or more metal cations. Among these cations, silver and manganese are preferred.

US—A—3720723 requires a co-precipitation of a catalyst and a co-catalyst. This co-precipitate needs a Group 2B metal besides copper. As the Group 2B metal zinc is preferred but chlorides of cadmium and mercury can be employed as well. However, a support is not essential and can be omitted.

GB—A—1263806 uses a catalyst prepared by exchange of alkaline earth or other metal ions of a 13X molecular sieve support by other metals.

Here again the metal ions are exchanged, i.e. they are chemically bonded.

WO—A—1—80/02023 requires that "a substantial part, at least of the copper should be chemically bonded to the support as opposed to being merely absorbed onto the support as an adsorbed copper salt, e.g., copper chloride. The type of the chemical bond is explained as being ionically into the support as a result of ion-exchange.

It is generally known that saturated compounds may be oxyhalogenated. A primary drawback of known processes for the oxyhalogenation of saturated compounds is their inability to selectively produce compounds which are less than fully halogenated. Thus, an attempt to produce 1,2-dichloroethane from saturated compounds via known oxychlorination processes typically results in a product mixture which contains a full spectrum of chlorinated compounds which typically are fairly difficult to separate. Another drawback of said oxychlorination processes is their high operating temperatures. High temperatures are conducive to the cracking of 1,2-dichloroethane to produce chloroethane (vinyl chloride) in the product. The presence of chloroethene in the product stream further complicates the isolation of 1,2-dichloroethane. Additionally, a high temperature process is typically more expensive to operate than a process operated at a lower temperature.

It would be advantageous to have a process for the production of 1,2-dichloroethane and related compounds which could use a starting material, e.g., a saturated compound, having a lower energy input than, for example, ethene. It also would be advantageous to have a process for the production of 1,2-dichloroethane and related compounds from saturated compounds which could be operated at a lower temperature than conventional processes for the oxychlorination of saturated compounds, and, perhaps more importantly, which could produce 1,2-dichloroethane and related compounds with very high selectivity.

Heretofore, a highly selective catalytic process for the preparation of 1,2-dichloroethane from chloroethane has not been disclosed. Such a process would be advantageous in that chloroethane could be prepared from ethane; therefore, 1,2-dichloroethane could be produced without using ethene (the production of which is a very energy intensive process) as a precursor.

In one aspect, the present invention is a process of selectively preparing saturated dihalohydrocarbons by contacting oxygen, a hydrogen halide and a monosubstituted saturated lower hydrocarbon in the presence of a supported catalyst of this invention under relatively mild conditions. Surprisingly, the practice of said process requires only one step to produce the desired product at relatively low temperatures with very high selectivities.

In another aspect, this invention is the discovery of novel catalyst systems having a variable valence

2

metal compound by an incipient wetting procedure predominantly on the surface of a zeolitic support, the zeolitic support having a maximum average pore diameter of 60 nm. These catalyst systems promote the highly selective conversion of saturated mono-substituted hydrocarbons to saturated dihalohalohydrocarbons at unexpectedly low temperatures.

The monosubstituted saturated lower hydrocarbons of the present invention are saturated hydrocarbons having one substituent which is not hydrogen. Preferably, these compounds have 2—8 carbon atoms, and more preferably, 2—6 carbon atoms. Suitable non-hydrogen substituents include OH, Cl, Br, F, and I. The preferred non-hydrogen substituents are OH and Cl, with Cl being most preferred. Suitable monosubstituted saturated lower hydrocarbons include chloroethane (ethyl chloride), ethanol bromoethane, 1-chloropropane, 2-chloropropane and the like. Preferred monosubstituted saturated lower hydrocarbons are chloroethane and ethanol, with chloroethane being most preferred. Other materials which may be oxyhalogenated with the catalyst of the present invention include saturated and unsaturated lower hydrocarbons such as ethane, propane, butane, ethene (ethylene), propene, 1-butene, 2-butene, and the like.

The feed gases may be used in any ratios which give the desired products. Typically, molar ratios of oxygen to monosubstituted saturated lower hydrocarbons of from about 0.1 to about 5 are employed, and molar ratios of hydrogen halide to monosubstituted saturated lower hydrocarbons of from about 0.1 to about 4 are employed. Nitrogen, which is preferred, or other inert gases may be employed to dilute the feed gas stream. The inert gas may be employed in any practical ratio, although care should be taken to avoid explosive mixtures. Any suitable source of oxygen may be employed, including air, commercially pure oxygen and the like. The hydrogen halide typically is chosen so that the halide is the same as the halogen substituents on the saturated dihalohydrocarbon product. Hydrogen chloride is the preferred hydrogen halide.

The novel catalysts of the present invention are compositions having a variable valence metal compound by an incipient wetting procedure predominantly on the surface of a zeolitic support. Suitable variable valence metals include, for example, copper and iron. Copper is the preferred variable valence metal. The original anion of the variable valence metal compound is immaterial, since said anion will be replaced with a halogen during the oxyhalogenation process to form the active catalyst. For example, in an oxychlorination process hydrogen chloride will contact the original variable valence metal compound and a chloride compound will be formed and will be the active catalyst. Chloride is the preferred anion for the variable valence metal compound for oxychlorination reactions.

Copper chloride is the preferred variable valence metal compound for use in oxychlorinations. Copper chloride is usually employed as cupric chloride, but it may be employed as cuprous chloride or mixtures of both.

The zeolitic supports of the present invention are zeolites having a maximum average pore diameter of 0.6 nm (nm=nanometers ($10^{-9}$ meters)). Suitable zeolitic supports include synthetic siliceous zeolites such as, for example, silicalite, ZSM-5, ZSM-8, ZSM-11 and the like. Preferred zeolitic supports have an average pore diameter of about 0.5 to 0.6 nm. The preferred zeolitic supports are silicalite and ZSM-5 with silicalite being most preferred.

The synthetic siliceous zeolites employed in the present invention are well-known in the art. ZSM-5 has been described in US Patent 3,702,886. Silicalite is further described as crystalline silica which after calcination in air at 600°C for one hour produces a silica polymorph having a mean refractive index of 1.39±0.01 and a specific gravity at 25°C of 1.70±0.05 g/cc. Silicalite has been described in US Patent 4,061,724. D. H. Olson et al., writing in *J. of Catalysis*, 61, 390—396 (1980) clarified the various zeolite structures related to ZSM-5 and concluded that highly siliceous pentasil structures such as silicalite have properties in conformity with and directly related to the level of aluminum content. Therefore, silicalite may be considered as an end member of a substitutional series, e.g., a substantially aluminum-free form of ZSM-5. For the above teachings, these references are herein incorporated by reference in their entireties. The zeolites typically are employed in either an alkali metal or hydrogen ion form.

The catalyst of the present invention is prepared by depositing the variable valence metal compound onto the zeolitic support. This is done by applying a solution of the variable valence metal compound to the zeolitic support. The solution may be applied by any method which wets the porous support to the point of incipient wetness. For example, the technique termed "dry impregnation" or "impregnation to incipient wetness", which is referred to in *Heterogeneous Catalysis in Practice*, by C. N. Satterfield (1980) at pp. 82—83, may be used to prepare the catalyst. Typically, the solution is sprayed or sprinkled onto the zeolitic support. No additional special processing or preparation of the catalyst is required other than normal procedures such as drying to remove the solvent of the solution, and calcining in order to remove organic residues.

Advantageously, the catalyst contains a catalytic amount of the variable valence metal compound. Typically, the amount of variable valence metal compound used is such that the catalyst contains between about 0.5 and about 20 weight percent of the variable valence metal, calculated as the uncombined metal, based on the dried weight of the zeolitic support.

The catalyst is generally placed in a reaction zone in such fashion as to allow the rapid passage of vapor or gas therethrough. The catalyst in the reaction zone may be either a fixed bed or a fluidized bed.

The oxyhalogenation of the present invention is advantageously conducted at a temperature of from about 200°C to about 400°C, preferably from about 225°C to about 325°C. Temperatures lower than 200°C or higher than 400°C may be employed; however, optimum results are obtained within the temperature range given above. Ordinarily, the reaction will proceed at atmospheric pressure or higher, but subatmospheric pressures may be employed if desired.

Periodically, the reactor may be shut down and the carbon or coke formation, if any, removed. This is usually accomplished by burning off, that is, heating the reactor in the presence of oxygen or air. Usually a comparatively mild temperature in the range of about 250°C to about 400°C is sufficient to remove the coke formation.

The reaction or contact time of the mono-substituted saturated lower hydrocarbon with the catalyst in the reactor can be varied. The contact time necessary between the monosubstituted saturated lower hydrocarbon and catalyst to promote the desired oxyhalogenation reaction is obtained by controlling the space velocity of the gaseous material passing through the reaction zone. The contact time is dependent upon several factors, namely, the scale of the operation, the quantity of catalyst in the reactor, and the type of reactor employed. For most reactors a contact time as high as about 25 seconds or more and as low as 0.5 second can be employed. If the contact time is too low the quantity of unreacted monosubstituted saturated lower hydrocarbon coming over is too high. On the other hand, if the contact time is too high, that is, much above 25 seconds, the impurities increase which makes it more difficult to recover the desired compound in a pure form. One can readily adjust the gaseous feed rate to obtain the optimum reaction or contact time for any particular type reactor.

Feed rates of the various components may be individually adjusted so as to provide maximum selectivity to the desired product with respect to the particular apparatus employed, catalyst concentration, pressure and temperature.

When the monosubstituted saturated lower hydrocarbon contacts the catalyst under the conditions described hereinbefore, the monosubstituted saturated lower hydrocarbon is converted selectively into a saturated dihalohydrocarbon. The gaseous mixture that is withdrawn from the reaction zone can be passed directly to a condenser thereby recovering the condensable materials while allowing the non-condensables, such as oxygen and hydrogen halide, to pass overhead and be recycled. The condensables may be separated into the desired dihalogenated product and recyclable materials.

The following examples are given to illustrate the invention, but are not to be construed as limiting its scope. All parts and percentages are by weight unless otherwise indicated.

Example 1

A copper chloride impregnated catalyst is prepared using the incipient wetness technique. Pellets of Linde molecular sieve S115 silicalite (lot 8251—1—2) containing 15 percent silica as a binder is crushed and sieved to a size of 7—12 mesh. A solution is prepared by dissolving 60 g of $CuCl_2 \cdot 2H_2O$ in 100 cc of water. A 6-g sample is placed in a glass cylindrical vessel and is wetted slowly with the solution until no more solution is drawn into the porous particles. The particles are then removed from the vessel and are contacted briefly with a paper towel in order to remove excess liquid from the surface of the particles. The wetting process uses 5 cc of the solution. The wetted catalyst, having a mass of 10.7 g, is dried at a temperature of 125°C for approximately 2 hours. The dried catalyst has a mass of 7 g.

A 6.3-g sample of the dried catalyst is placed in a glass reaction vessel. The vessel has a nominal outside diameter of $\frac{1}{2}$ inch (13 mm) and is equipped with a means for uniform heating of the outer wall, and a temperature measuring means which is in a well in the center of the bed of catalyst. The bed of catalyst in the reaction vessel has a height of 4.25 inches (108 mm). The reaction vessel is heated to a temperature of 220°C overnight in order to calcine the catalyst.

Examples 2—4

A number of reactions are performed and the results are summarized in Table I. For each reaction, a 21-stage static mixer is used to thoroughly mix the reactants, all of which are in the gas phase, to form a gaseous mixture. Individual gas flow rates are measured and controlled by calibrated flow meters. A gas chromatograph having thermal conductivity and flame ionization detectors is employed to determine the composition of the gaseous mixture before and after it passes through the catalyst bed.

**0 106 356**

TABLE I

| | Ex. 2 | Ex. 3 | Ex. 4 |
|---|---|---|---|
| $O_2$ cc/min | 10 | 15 | 10 |
| air cc/min | 15 | — | 15 |
| $N_2$ cc/min | — | 25 | — |
| HCl cc/min | 7 | | |
| ethanol cc/min | — | — | 9.3 |
| chloroethane cc/min | 11 | 12 | — |
| Temperature °C | 255 | 275 | 255 |
| Conversion of chloroethane (volume %) | 45% | 60% | — |
| Conversion of ethanol (volume %) | — | — | 90+% |
| Reactor effluent stream organic analysis (volume %) chloroethane | 55 | 40 | 59 |
| ethene | 6 | 16 | 13 |
| 1,2-dichloroethane | 38 | 43 | 27 |
| $CO_2$ | * | * | * |
| (cis and trans) 1,2-dichloroethane | * | * | * |
| 1,1,2-trichloroethane | * | * | * |
| 1,1,2,2-tetrachloroethane | * | * | * |

*Trace amount, sum of trace amounts is less than 1 volume percent for each Example.

It may be observed from Table I that the catalyst and process of the present invention provide conversion of monosubstituted saturated lower hydrocarbons to saturated dichlorohydrocarbons with exceptionally high selectivity, as the total amount of by-products produced is less than one volume percent of the product stream in each case.

**Claims**

1. A catalyst composition consisting essentially of a variable valence metal compound deposited by an incipient wetting procedure predominantly on the surface of a zeolitic support having a maximum average pore diameter of 0.6 nm.

2. A catalyst composition according to Claim 1 wherein the variable valence metal compound is a copper halide.

3. A catalyst composition according to Claim 2 wherein the zeolitic support has an average pore diameter of 0.5 to 0.6 nm.

4. A catalyst composition according to Claim 2 wherein the copper halide is cupric chloride.

5. A catalyst composition according to Claim 4 wherein the support is silicalite.

6. A catalyst composition according to Claim 4 wherein the support is ZSM-5.

7. A process for converting a monosubstituted saturated lower hydrocarbon to a saturated dihalohydrocarbon comprising contacting in the vapor phase, oxygen, a hydrogen halide, and a monosubstituted saturated lower hydrocarbon having a non-hydrogen substituent selected from the group consisting of OH, Cl, Br, F, and I, in the presence of a catalytic amount of a catalyst composition consisting essentially of a variable valence metal compound deposited by an incipient wetting procedure predominantly on the surface of a zeolitic support, the support having a maximum average pore diameter of 0.6 nm; under reaction conditions such that a saturated dihalohydrocarbon is produced.

5

8. A process of Claim 7, wherein the zeolitic support has an average pore diameter of about 0.5 to 0.6 nm.

9. The process of Claim 8 wherein the process temperature is from 200°C to 400°C.

10. A process of Claim 8 wherein the variable valence metal compound is copper chloride.

11. A process of Claim 10 wherein the monosubstituted saturated lower hydrocarbon has from 2 to 6 carbon atoms and the non-hydrogen substituent is OH or Cl.

12. A process of Claim 11 wherein the nonhydrogen substituent is Cl.

13. A process of Claim 12 wherein the monosubstituted saturated lower hydrocarbon is chloroethane, the saturated dihalohydrocarbon is 1,2-dichloroethane, and the hydrogen halide is hydrogen chloride.

14. A process for preparing 1,2-dichloroethane comprising contacting in the vapor phase, chloroethane, oxygen, and hydrogen chloride, in the presence of a catalytic amount of a composition consisting essentially of copper chloride deposited by an incipient wetting procedure predominantly on the surface of a zeolitic support, said support having a maximum average pore diameter of 0.6 nm; under reaction conditions such that 1,2-dichloroethane is produced.

**Patentansprüche**

1. Katalysatorzusammensetzung, bestehend im wesentlichen aus einer variablen Valenzmetallverbindung, die mittels einem inzipienten Benetzungsverfahren im wesentlichen auf der Oberfläche eines Zeolittrögers aufgebracht ist, der einen maximalen durchschnittlichen Porendurchmesser von 0,6 nm aufweist.

2. Katalysatorzusammensetzung nach Anspruch 1, bei der die variable Valenzmetallverbindung ein Kupferhalogenid ist.

3. Katalysatorzusammensetzung nach Anspruch 2, bei der der Zeolittröger einen durchschnittlichen Porendurchmesser von 0,5 bis 0,6 nm aufweist.

4. Katalysatorzusammensetzung nach Anspruch 2, bei der das Kupferhalogenid Kupfer(II)chlorid ist.

5. Katalysatorzusammensetzung nach Anspruch 4, bei der der Träger Silikalit ist.

6. Katalysatorzusammensetzung nach Anspruch 4, bei der der Träger ZSM-5 ist.

7. Verfahren zur Überführung eines monosubstituierten gesättigten niedrigen Kohlenwasserstoffs in einen gesättigten Dihalo-Kohlenwasserstoff, umfassend das Inkontaktbringen in der Dampfphase von Sauerstoff, einem Wasserstoffhalogenid und einem monosubstituierten gesättigten niedrigen Kohlenwasserstoff, der einen Substituenten hat, der nicht Wasserstoff ist und der ausgewählt ist aus der Gruppe umfassend OH, Cl, Br, F und J, in Gegenwart einer katalytischen Menge einer Katalysatorzusammensetzung, bestehend im wesentlichan aus einer variablen Valenzmetallverbindung, die mittels einem inzipienten Benetzungsverfahren im wesentlichen auf der Oberfläche eines Zelitträgers abgelagert ist, wobei der Träger einen maximalen Durchschnittsporendurchmesser von 0,6 nm aufweist; unter solchen Reaktionsbedingungen, daß ein gesättigter Dihalogenkohlenwasserstoff produziert wird.

8. Verfahren nach Anspruch 7, bei dem der Zeolittröger einen durchschnittlichen Porendurchmesser von etwa 0,5 bis 0,6 nm aufweist.

9. Verfahren nach Anspruch 8, bei dem die Verfahrenstemperatur zwischen 200°C und 400°C liegt.

10. Verfahren nach Anspruch 8, bei dem die variable Valenzmetallverbindung Kupferchlorid ist.

11. Verfahren nach Anspruch 10, bei dem der monosubstituierte gesättigte niedrige Kohlenwasserstoff 2 bis 6 Kohlenstoffatome aufweist und der Substituent, der kein Wasserstoff ist, OH oder Cl ist.

12. Verfahren nach Anspruch 11, bei dem der Substituent, der kein Wasserstoff ist, Chlor ist.

13. Verfahren nach Anspruch 12, bei dem der monosubstituierte gesättigte niedrige Kohlenwasserstoff Chlorethan, der gesättigte Dihalogenkohlenwasserstoff 1,2-Dichlorethan und der Halogenwasserstoff Salzsäure ist.

14. Verfahren zur Herstellung von 1,2-Dichlorethan, umfassend das Inkontaktbringen in der Dampfphase von Chlorethan, Sauerstoff und Salzsäure in Gegenwart einer katalytischen Menge einer Zusammensetzung, die im wesentlichen aus Kupferchlorid besteht, das mittels einem inzipienten Benetzungsverfahren im wesentlichen auf der Oberfläche eines Zeolittrögers aufgebracht ist, wobei der Träger einen maximalen durchschnittlichen Porendurchmesser von 0,6 nm aufweist, unter solchen Reaktionsbedingungen, daß 1,2-Dichlorethan entsteht.

**Revendications**

1. Composition de catalyseur, consistant essentiellement en un composé d'un métal à valence variable, déposé, par un procédé d'humidification jusqu'à suintement, principalement à la surface d'un support zéolitique ayant un diamètre moyen maximal de pores de 0,6 nm.

2. Composition de catalyseur selon la revendication 1, dans laquelle le composé de métal à valence variable est un halogénure de cuivre.

3. Composition de catalyseur selon la revendication 2, dans laquelle le support zéolitique présente un diamètre moyen de pores de 0,5 à 0,6 nm.

4. Composition de catalyseur selon la revendication 2, dans laquelle l'halogénure de cuivre est le chlorure cuivrique.

5. Composition de catalyseur selon la revendication 4, dans laquelle le support est de la silicalite.

6. Composition de catalyseur selon la revendication 4, dans laquelle le support est de la ZSM-5.

7. Procédé de conversion d'un hydrocarbure inférieur saturé monosubstitué en un dihalogénohydrocarbure saturé, qui comprend la mise en contact, en phase vapeur, d'oxygène, d'un halogénure d'hydrogène et d'un hydrocarbure inférieur saturé monosubstitué portant un substituant, autre qu'un hydrogène, choisi dans le groupe constitué de OH, Cl, Br, F et I, en présence d'une quantité catalytique d'une composition de catalyseur constituée essentiellement d'un composé d'un métal à valence variable déposé, par un procédé d'humidification jusqu'à suintement, principalement à la surface d'un support zéolitique, ce support présentant un diamètre moyen maximal de pore de 0,6 nm; et ce, dans des conditions de réaction telles qu'un obtient un dihalogénohydrocarbure saturé.

8. Procédé selon la revendication 7, dans lequel le support zéolitique présente un diamètre moyen de pores d'environ 0,5 à 0,6 nm.

9. Procédé selon la revendication 8, dans lequel la température opératoire est de 200 à 400°C.

10. Procédé selon la revendication 8, dans lequel le composé de métal à valence variable est un chlorure de cuivre.

11. Procédé selon la revendication 10, dans lequel l'hydrocarbure inférieur saturé monosubstitué comporte de 2 à 6 atomes de carbone et le substituant autre que l'hydrogène est OH ou Cl.

12. Procédé selon la revendication 11, dans lequel le substituant autre que l'hydrogène est Cl.

13. Procédé selon la revendication 12, dans lequel l'hydrocarbure inférieur saturé monosubstitué est le chloroéthane, le dihalogénohydrocarbure saturé est le 1,2-dichloroéthane et l'halogénure d'hydrogène est le chlorure d'hydrogène.

14. Procédé de préparation du 1,2-dichloroéthane, qui comprend la mise en contact en phase vapeur, de chloroéthane, d'oxygène et de chlorure d'hydrogène, en présence d'une quantité catalytique d'une composition constituée essentiellement de chlorure de cuivre déposé, par un procédé d'humidification jusqu'à suintement, principalement à la surface d'un support zéolitique, ledit support ayant un diamètre moyen maximal de pores de 0,6 nm; et ce, dans des conditions de réaction telles qu'on obtient du 1,2-dichloroéthane.